# EUROPEAN PATENT APPLICATION

(11) **EP 4 442 324 A1**
(43) Date of publication of application: **09.10.2024**
(21) Application number: 22901117.6
(22) Date of filing: 18.11.2022
(51) Int. Cl.: A61Q 1/00, A61Q 17/04, A61Q 19/00, A61K 8/49, A61K 8/9761

(54) **COSMETIC**

(30) Priority: 02.12.2021 JP 2021196461
(71) Applicant: Shiseido Company, Ltd., Chuo-ku Tokyo 104-0061 (JP)
(72) Inventor: NODA Seigi, Tokyo 104-0061 (JP); OKUYAMA Yusuke, Tokyo 104-0061 (JP); SUZUKI Ikuhiro, Tokyo 104-0061 (JP)
(74) Representative: Ter Meer Steinmeister & Partner
(86) International application number: PCT/JP2022/042810
(87) International publication number: WO 2023/100684

(57) **Abstract**

[Problem] To provide a cosmetic excellent in storage stability, according to the present invention.

[Solution] A cosmetic including (A) a Thuja orientalis seed extract, (B) a cyclic carboxamide derivative having a specific structure or its salt, and (C) water.

## Description

### TECHNICAL FIELD

The present invention relates to a cosmetic including a Thuja orientalis seed extract.

### BACKGROUND ART

A Thuja orientalis seed extract is a herbal medicine obtained by drying a seed of Platycladus orientalis Franco, Thuja orientalis L., or Biota orientalis Endl. of Cupressaceae. A Thuja orientalis seed extract has a melanin generation inhibitory effect, and is used as an effective whitening component in a whitening cosmetic. Various methods for preparing a Thuja orientalis seed extract have also been proposed (for example, Patent Literature 1).

Meanwhile, a cyclic carboxamide derivative is known to have an anti-wrinkle effect and a pigmentation inhibitory effect, and is proposed to be blended in a cosmetic and the like (for example, Patent Literature 2).

### CITATION LIST

### PATENT LITERATURE

Patent Literature 1: WO 2012/057123 A
Patent Literature 2: WO 2011/040496 A

### SUMMARY OF THE INVENTION

According to the studies of the present inventors, it has been found that a Thuja orientalis seed extract may generate an unpleasant odor in a high-temperature environment. Then, it has been surprisingly found that generation of an unpleasant odor can be suppressed by combining a specific cyclic carboxamide derivative with a Thuja orientalis seed extract. The present invention is based on these findings.

According to the present invention, the following invention is provided.
[1] A cosmetic including:
   (A) a Thuja orientalis seed extract;
   (B) a cyclic carboxamide derivative or a salt of the cyclic carboxamide derivative, the cyclic carboxamide derivative represented by Formula (1): 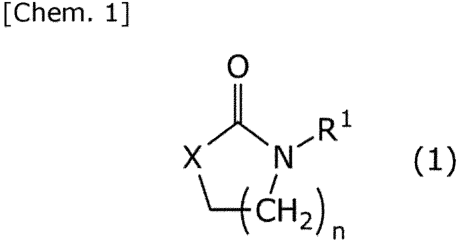 wherein
      R¹ represents a hydrocarbon group having 1 to 6 carbon atoms optionally substituted with a hydroxy group or R¹ represents a hydrogen atom,
      X represents -CH₂- or -N(R²)- wherein R² represents a hydrocarbon group having 1 to 6 carbon atoms optionally substituted with a hydroxy group or R² represents a hydrogen atom, and
      n is an integer of 1 to 3; and
   (C) water.
[2] The cosmetic according [1], wherein
   in Formula (1) of the component (B),
   R¹ represents a hydroxyalkyl group having 1 to 3 carbon atoms,
   X represents -CH₂- or -NH-, and
   n is 1.
[3] The cosmetic according to [1] or [2], wherein the component (B) is 1-(2-hydroxyethyl)-2-imidazolidinone.
[4] The cosmetic according to any one of [1] to [3], wherein an amount of the component (B) blended is 0.01 to 8 mass% with respect to a total amount of the cosmetic.
[5] The cosmetic according to any one of [1] to [4], wherein the component (A) is a hydrolyzed Thuja orientalis seed extract.
[6] The cosmetic according to any one of [1] to [5], wherein an amount of the component (A) blended is 0.01 to 3 mass% with respect to the total amount of the cosmetic.
[7] The cosmetic according to any one of [1] to [6], further including a water-soluble thickener.

According to the present invention, a cosmetic excellent in storage stability can be provided. The present invention can particularly suppress generation of an unpleasant odor derived from a Thuja orientalis seed extract in a high-temperature environment.

### BEST MODE FOR CARRYING OUT THE INVENTION

The present invention relates to a cosmetic including (A) a Thuja orientalis seed extract, (B) a cyclic carboxamide derivative having a specific structure or its salt, and (C) water.

### (A) Thuja Orientalis Seed Extract

The cosmetic according to the present invention includes (A) a Thuja orientalis seed extract (hereinafter, sometimes referred to as a component (A), and the same applies to other components).

As the Thuja orientalis seed used in the present invention, the seed kernel (endosperm part) is preferably used, but the entire seed can also be used because the active ingredient is also contained in the entire seed. The Thuja orientalis seed extract may be produced with a conventional method, or a commercially available product can be used.

The method for preparing the Thuja orientalis seed extract can be, but is not limited to, a known method of production. Examples of the method for preparing the Thuja orientalis seed extract include a method in which a plant extract extracted from a plant seed is subjected to an alkali treatment (hereinafter, referred to as a first method), and a method in which a plant seed is ground, aged at a temperature of 10 to 35°C and a relative humidity of 20 to 90% for 1 week or more, and then subjected to extraction (hereinafter, referred to as a second method).

The extraction solvent used in the first method and the second method may be any volatile solvent usually used for extraction, and in particular, alcohols such as methanol and ethanol, water-containing alcohols, polar or nonpolar organic solvents such as acetone, ethyl acetate, hexane, and ether can be used singly or in combination, but acetone, ethyl acetate, and hexane are particularly preferable from the viewpoint of inexpensiveness and ease of concentration under reduced pressure. In addition, extraction with a supercritical carbon dioxide gas can also be used. On the other hand, an extraction solvent containing a large amount of water is not preferable because extraction of the active ingredient is suppressed.

The extraction is performed for a certain period with a solvent having a mass about 1 to 100 times, preferably about 1 to 30 times the mass of the seed. The seed to be used can be appropriately ground as necessary, but may be used in an unground state in a case where, for example, clogging may cause a problem at the time of filtration. Alternatively, extraction can be performed repeatedly with a small amount of solvent, and a reflux apparatus such as a Soxhlet extractor may be used. In the case of extraction with a supercritical carbon dioxide gas, extraction can be performed under general conditions of around 40°C and 20 to 40 MPa.

In the first method, the extraction solvent is removed from the extract, and then the extract is subjected to an alkali treatment and thus hydrolyzed. In the alkali treatment, a concentrated alkali aqueous solution having a volume 0.2 to 2 times the volume of the extract is added to the extract after removing the solvent, and then the mixture is sufficiently stirred and mixed, and aged for about 30 minutes to several days. The temperature at the time of mixing is preferably in a range of room temperature to 70°C, and more preferably in a range of 30 to 60°C. At the time of aging, appropriate mixing is desirably performed so as not to separate the hydrophilic component and the lipophilic component. Examples of the concentrated alkali aqueous solution include aqueous solutions containing NaOH, KOH or the like at a concentration of 1 to 10 N.

When the extract hydrolyzed by the alkali treatment as described above is further neutralized with an acid to be nearly neutral, an oily substance is separated. This oily substance exhibits high safety to the skin while exhibiting an extremely high dermal pigmentation inhibitory effect. In order to promote the recovery of the oily substance, an alcohol, acetone, or the like may be appropriately added after the alkali treatment, and then operations such as decoloring, deodorization, desalting, and distillation may be applied as necessary.

In the second method, the seed is appropriately ground or pressed before extraction, and is aged as it is for a certain period at around room temperature. The aging period is not particularly limited as long as an intended effect can be obtained, but an aging period as short as one week or less makes it difficult to obtain a sufficient whitening effect, and meanwhile, an aging period as long as several years causes problems of decay and change in an odor by oxidation, and thus such aging periods are not preferable. During aging, change in an odor can be also suppressed by addition of an antioxidant, substitution with nitrogen, or the like as necessary.

The seed thus aged is subjected to extraction using the extraction solvent and the extraction method described above. After the extraction, operations such as solvent removal, decoloring, deodorization, and distillation are performed as necessary. Such an extract obtained by grinding and aging a specific seed and then performing extraction exhibits high safety to the skin while exhibiting a dermal pigmentation inhibitory effect like the above-described alkali-treated extract.

The Thuja orientalis seed extract prepared with the first method is also referred to as a hydrolyzed Thuja orientalis seed extract. The component (A) is preferably a hydrolyzed Thuja orientalis seed extract.

The amount of the component (A) blended is preferably 0.005 to 3 mass%, more preferably 0.01 to 3 mass%, and still more preferably 0.1 to 1.5 mass% with respect to the total amount of the cosmetic.

### (B) Cyclic Carboxamide Derivative or Its Salt

The cosmetic according to the present invention includes (B) a cyclic carboxamide derivative represented by Formula (1) or its salt.

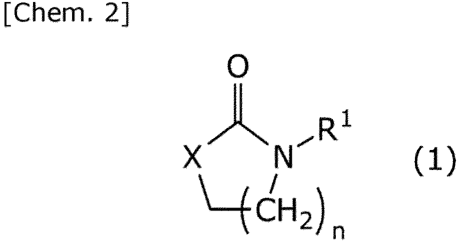

In Formula (1),
R¹ represents a hydrocarbon group having 1 to 6 carbon atoms optionally substituted with a hydroxy group or R¹ represents a hydrogen atom,
X represents -CH₂- or -N(R²)- wherein R² represents a hydrocarbon group having 1 to 6 carbon atoms optionally substituted with a hydroxy group or R² represents a hydrogen atom, and
n is an integer of 1 to 3

The hydrocarbon group is not particularly limited, and may be, for example, an alkyl group, a cycloalkyl group, an alkenyl group, an alkynyl group, a cycloalkylalkyl group, a haloalkyl group, an alkoxyalkyl group, or an alkoxycarbonylalkyl group, and is preferably an alkyl group.

In a preferred embodiment, in Formula (1) of the component (B),
R¹ represents a hydroxyalkyl group having 1 to 3 carbon atoms,
X represents -CH₂- or -NH-, and
n is 1.

Specific examples of the cyclic carboxamide derivative represented by Formula (1) include the following.

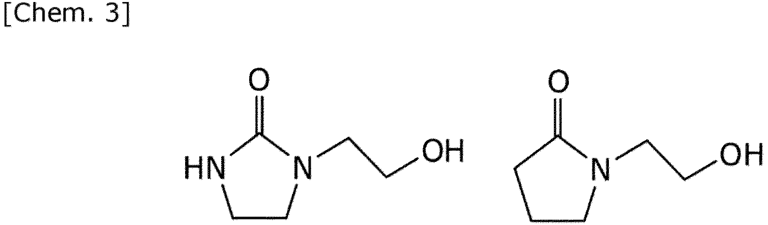

The component (B) is most preferably 1-(2-hydroxyethyl)-2-imidazolidinone.

The component (B) may be a salt of the cyclic carboxamide derivative represented by Formula (1). The kind of the salt is not particularly limited as long as the salt is pharmacologically acceptable, and the salt may be an inorganic salt or an organic salt. Examples of the inorganic salt include a hydrochloride, a sulfate, a phosphate, a hydrobromide, a sodium salt, a potassium salt, a magnesium salt, a calcium salt, a magnesium salt, and an ammonium salt. Examples of the organic salt include an acetate, a lactate, a maleate, a fumarate, a tartrate, a methanesulfonate, a p-toluenesulfonate, a triethanolamine salt, and an amino acid salt.

One or more kinds of the component (B) can be blended. The amount of the component (B) blended is preferably 0.01 to 8 mass%, more preferably 0.1 to 8 mass%, and still more preferably 0.8 to 8 mass% with respect to the total amount of the cosmetic.

### (C) Water

The cosmetic according to the present invention includes (C) water. As the water, water used in cosmetics, quasi-drugs, and the like can be used, and for example, purified water, ion-exchanged water, tap water, or the like can be used.

The amount of the water blended is preferably 40 to 95 mass%, and more preferably 50 to 85 mass% with respect to the total amount of the cosmetic according to the present invention.

### (D) Water-Soluble Thickener

The cosmetic according to the present invention can further include (D) a water-soluble thickener.

Examples of the component (D) include plant-based polymers such as gum arabic, tragacanth gum, galactan, carob gum, guar gum, karaya gum, carrageenan, pectin, agar, quince seed (Pyrus cydonia), and ALGAE COLLOID (Phaeophyta extract), microbial polymers such as dextran, succinoglucan, pullulan, and xanthan gum, animal-based polymers such as collagen, casein, albumin, and gelatin, cellulose-based polymers such as methylcellulose, nitrocellulose, ethylcellulose, methylhydroxypropylcellulose, hydroxyethylcellulose, sodium cellulose sulfate, hydroxypropylcellulose, carboxymethylcellulose sodium, crystalline cellulose, and cellulose powder, alginic acid-based polymers such as sodium alginate and propylene glycol alginate, vinyl-based polymers such as polyvinyl alcohol, polyvinyl methyl ether, polyvinylpyrrolidone, and a carboxyvinyl polymer (carbomer), polyoxyethylene-based polymers such as hydrophobically modified polyetherurethane, acrylic polymers such as sodium polyacrylate, polyethyl acrylate, and polyacrylamide, and inorganic water-soluble polymers such as polyethyleneimine, cationic polymers, bentonite, aluminum magnesium silicate, laponite, hectorite, and silicic anhydride.

Among them, acrylic polymers are preferable. In particular, an acrylic acid-alkyl methacrylate copolymer (alkyl-modified carboxyvinyl polymer) is preferable, and an acrylates/C10-30 alkyl acrylate crosspolymer is more preferable because such a polymer has surface activity and also has an effect of preventing aggregation and coalescence of large particles. As the acrylates/C10-30 alkyl acrylate crosspolymer, a commercially available product may be used, and examples of the product include "CARBOPOL 1342", "Pemulen TR-1 Polymeric Emulsifier", and "Pemulen TR-2 Polymeric Emulsifier" (by Lubrizol Advanced Materials, Inc.).

As the acrylic polymer, for example, a (sodium acrylate/sodium acryloyldimethyltaurate) copolymer, a (dimethylacrylamide/sodium acryloyldimethyltaurate) crosspolymer, an (ammonium acryloyldimethyltaurate/beheneth-25 methacrylate) crosspolymer, or the like can also be used.

One or more kinds of the component (D) can be blended. The amount of the component (D) blended is preferably 0.01 to 5 mass%, and more preferably 0.1 to 1 mass% with respect to the total amount of the cosmetic.

### (E) Oil

The cosmetic according to the present invention can further include (E) an oil. Examples of the component (E) include hydrocarbon oils, silicone oils, ester oils, higher fatty acids, liquid oils, solid oils, and semi-solid oils, and the component (E) is preferably selected from the group consisting of hydrocarbon oils, silicone oils, and ester oils.

Examples of the hydrocarbon oils include isododecane, isohexadecane, isoparaffin, mineral oil (liquid paraffin), ozokerite, squalane, pristane, paraffin, ceresin, squalene, petrolatum, microcrystalline wax, and hydrogenated polydecene.

Examples of the silicone oils include chain polysiloxanes (such as dimethicone, diphenylsiloxy phenyl trimethicone, and diphenylpolysiloxane), cyclic polysiloxanes (such as octamethylcyclotetrasiloxane, decamethylcyclopentasiloxane, and dodecamethylcyclohexasiloxane), silicone resins forming a three-dimensional network structure, silicone rubber, various modified polysiloxanes (such as an amino-modified polysiloxane, a polyether-modified polysiloxane, an alkyl-modified polysiloxane, and a fluorine-modified polysiloxane), and acrylic silicones, and chain polysiloxanes are preferable.

Examples of the ester oils include octyl octanoate, nonyl nonanoate, cetyl octanoate, isopropyl myristate, octyldodecyl myristate, isopropyl palmitate, ethylhexyl palmitate, butyl stearate, hexyl laurate, myristyl myristate, decyl oleate, hexyldecyl dimethyl octanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkylglycol monoisostearate, neopentyl glycol dicaprate, tripropylene glycol pivalate, diisostearyl malate, glyceryl di-2-heptylundecanoate, glyceryl diisostearate, trimethylolpropane tri-2-ethylhexanoate, trimethylolpropane triisostearate, pentaerythrityl tetraethylhexanoate, glyceryl tri-2-ethylhexanoate (triethylhexanoin), glyceryl trioctanoate, glyceryl triisopalmitate, trimethylolpropane triisostearate, cetyl 2-ethylhexanoate-2-ethylhexyl palmitate, glyceryl trimyristate, glyceride tri-2-heptylundecanoate, castor oil fatty acid methyl ester, oleyl oleate, acetoglyceride, 2-heptylundecyl palmitate, diisobutyl adipate, N-lauroyl-L-glutamate-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, triethyl citrate, cetyl ethylhexanoate, and phytosteryl macadamiate.

Examples of the higher fatty acids include lauric acid, myristic acid, palmitic acid, stearic acid, behenic acid, oleic acid, undecylenic acid, tall oil acid, isostearic acid, linolic acid, linoleic acid, eicosapentaenoic acid (EPA), and docosahexaenoic acid (DHA).

Examples of the liquid oils include avocado oil, camellia oil, macadamia nut oil, corn oil, olive oil, rapeseed oil, sesame oil, persic oil, wheat germ oil, sasanqua oil, castor oil, linseed oil, safflower oil, cottonseed oil, perilla oil, soybean oil, peanut oil, tea seed oil, kaya oil, rice bran oil, paulownia oil, Japanese tung oil, jojoba oil, germ oil, and triglycerin oil. Examples of the solid oils include cacao butter, coconut oil, hardened coconut oil, palm oil, palm kernel oil, hydrogenated palm oil, Japan wax kernel oil, hardened oils, Japan wax, and hardened castor oil. Examples of the semi-solid oils include shea butter, partially hydrogenated coconut oil, and partially hydrogenated jojoba oil.

One or more kinds of the component (E) can be blended. The amount of the component (E) blended is preferably 0.1 to 30 mass%, and more preferably 0.5 to 20 mass% with respect to the total amount of the cosmetic.

In the cosmetic according to the present invention, an optional component usually used in cosmetics and pharmaceuticals can be blended in addition to the above-described components. As the optional component, for example, another component usually used in cosmetics, such as a moisturizer, a lower alcohol, a sequestering agent, a neutralizer, a pH adjuster, an antioxidant, a preservative, or a drug, can be blended, and one or more kinds thereof can be blended as long as an effect of the present invention is exhibited.

Examples of the moisturizer include polyethylene glycol, propylene glycol, dipropylene glycol (DPG), glycerin, 1,3-butylene glycol (BG), erythritol, xylitol, sorbitol, maltitol, chondroitin sulfate, hyaluronic acid, mucoitinsulfuric acid, charonin acid, atelocollagen, cholesteryl-12-hydroxystearate, sodium lactate, bile acid salts, dl-pyrrolidone carboxylates, short-chain soluble collagen, a chestnut rose extract, a yarrow extract, and a melilot extract.

Examples of the lower alcohol include ethanol, 1-propanol, 2-propanol, isobutyl alcohol, and t-butyl alcohol.

Examples of the sequestering agent include 1-hydroxyethane-1,1-diphosphonic acid, 1-hydroxyethane-1,1-diphosphonic acid tetrasodium salt, edetate disodium (EDTA-2Na), edetate trisodium, edetate tetrasodium, sodium citrate, sodium polyphosphate, sodium metaphosphate, gluconic acid, phosphoric acid, citric acid, succinic acid, edetic acid, and trisodium hydroxyethyl ethylenediamine triacetate.

Examples of the neutralizer include 2-amino-2-methyl-1-propanol, 2-amino-2-methyl-1,3-propanediol, potassium hydroxide, sodium hydroxide, triethanolamine, and sodium carbonate.

Examples of a pH adjuster include buffers such as lactic acid-sodium lactate, citric acid-sodium citrate, and succinic acid-sodium succinate.

Examples of the antioxidant include dibutylhydroxytoluene, butylhydroxyanisole, sodium pyrosulfite, and gallic acid esters.

Examples of the preservative include paraoxybenzoic acid esters such as methylparaben, ethylparaben, and butylparaben, benzoic acid, salicylic acid, sorbic acid, parachlorometacresol, hexachlorophene, benzalkonium chloride, chlorhexidine chloride, trichlorocarbanilide, photosensitizers, and phenoxyethanol.

Examples of the drug include ascorbic acid (vitamin C), tranexamic acid, kojic acid, ellagic acid, arbutin, alkoxysalicylic acid, glycyrrhizic acid, tocopherol, retinol, salts and derivatives thereof (for example, sodium L-ascorbate, L-ascorbic acid ester magnesium salt, L-ascorbic acid glucoside, 2-O-ethyl-L-ascorbic acid, 3-O-ethyl-L-ascorbic acid, 4-methoxysalicylic acid sodium salt, 4-methoxysalicylic acid potassium salt, dipotassium glycyrrhizinate, stearyl glycyrrhizinate, tocopherol acetate, retinol acetate, and retinol palmitate), nicotinic acid and its derivatives (for example, nicotinic acid amide), caffeine, tannin, verapamil and its derivatives, a licorice extract, glabridin, a hot water extract of Pyracantha fortuneana seed, various herbal medicines, hydrolyzed silk, hydrolyzed conchiolin, a tea extract, a Potentilla erecta root extract, an Angelica keiskei leaf/stem extract, an Aloe barbadensis leaf extract, a cherry leaf extract, an Angelica acutiloba Kitagawa root extract, a Citrus depressa peel extract, an Iris florentina root extract, an Eucheuma serra/Grateloupia sparsa/Saccharina angustata/Ulva linza/Undaria pinnatifida extract, a Typha angustifolia spike extract, an Isodonis japonicus leaf/stalk extract, a Camellia japonica seed extract, a Saccharina angustata/Undaria pinnatifida extract, a Bupleurum falcatum root extract, a Nasturtium officinale leaf/stem extract, a Cinnamomum cassia bark extract, a rosemary leaf oil, a lavender oil, glutamic acid, trimethylglycine, chlorphenesin, and menthoxypropanediol.

In addition, an ultraviolet absorber, a powder component, an aroma chemical, and the like can also be appropriately blended.

Examples of the form of the cosmetic according to the present invention include, but are not particularly limited to, forms of a solution, solubilization, emulsification, powder dispersion, a water 2-oil bilayer, and a water-oil-powder trilayer. One preferred embodiment is an oil-in-water cosmetic.

Examples of the cosmetic according to the present invention include skin care cosmetics (such as lotions, milky lotions, creams, beauty essences, and packs), makeup cosmetics (such as foundations and makeup bases), skin cleaning agents (such as face washes and makeup removers), sunscreen cosmetics, and ointments.

The cosmetic of the present invention can be produced in accordance with a conventional method. In a case where the cosmetic according to the present invention is an emulsion cosmetic, the method of emulsification is not particularly limited.

### [Examples]

The present invention will be described specifically with reference to the following Examples, but the present invention is not limited to these Examples. The content is shown in mass% with respect to the total amount unless otherwise specified.

### [Examples 1 to 9 and Comparative Examples 1 and 2]

Cosmetics of Examples 1 to 9 and Comparative Examples 1 and 2 were prepared at combinations shown in Table 1.

**[Table 1]**

| Table 1 | Example | | | | | | | | | Comparative | |
|---|---|---|---|---|---|---|---|---|---|---|---|
| | 1 | 2 | 3 | 4 | 5 | 6 | 7 | 8 | 9 | 1 | 2 |
| Hydrolyzed Thuja orientalis seed extract | 0.3 | 0.3 | 0.3 | 0.3 | 0.3 | 0.01 | 0.1 | 0.5 | 1 | 0.3 | 1 |
| 1-(2-Hydroxyethyl)-2-imidazolidinone | 0.5 | 1 | 1.5 | 3 | 5 | 1.5 | 1.5 | 1.5 | 1.5 | 0 | 0 |
| Water | Balance | Balance | Balance | Balance | Balance | Balance | Balance | Balance | Balance | Balance | Balance |
| Carbomer | 0.03 | 0.03 | 0.03 | 0.03 | 0.03 | 0.03 | 0.03 | 0.03 | 0.03 | 0.03 | 0.03 |
| (Acrylates/alkyl acrylate (C10-30)) crosspolymer | 0.07 | 0.07 | 0.07 | 0.07 | 0.07 | 0.07 | 0.07 | 0.07 | 0.07 | 0.07 | 0.07 |
| (Ammonium acryloyldimethyltaurate/behen eth-25 methacrylate) crosspolymer | 0.25 | 0.25 | 0.25 | 0.25 | 0.25 | 0.25 | 0.25 | 0.25 | 0.25 | 0.25 | 0.25 |
| Hydrogenated polydecene | 3 | 3 | 3 | 3 | 3 | 3 | 3 | 3 | 3 | 3 | 3 |
| Pentaerythrityl | 5 | 5 | 5 | 5 | 5 | 5 | 5 | 5 | 5 | 5 | 5 |
| Glycerin | 5 | 5 | 5 | 5 | 5 | 5 | 5 | 5 | 5 | 5 | 5 |
| BG | 8 | 8 | 8 | 8 | 8 | 8 | 8 | 8 | 8 | 8 | 8 |
| Sodium metaphosphate | Appropr iate amount | Appropr iate amount | Appropr iate amount | Appropr iate amount | Appropr iate amount | Appropr iate amount | Appropr iate amount | Appropr iate amount | Appropr iate amount | Appropr iate amount | Appropr iate amount |
| Potassium hydroxide | Appropr iate amount | Appropr iate amount | Appropr iate amount | Appropr iate amount | Appropr iate amount | Appropr iate amount | Appropr iate amount | Appropr iate amount | Appropr iate amount | Appropr iate amount | Appropr iate amount |
| Phenoxyethanol | Appropr iate amount | Appropr iate amount | Appropr iate amount | Appropr iate amount | Appropr iate amount | Appropr iate amount | Appropr iate amount | Appropr iate amount | Appropr iate amount | Appropr iate amount | Appropr iate amount |
| Total | 100 | 100 | 100 | 100 | 100 | 100 | 100 | 100 | 100 | 100 | 100 |
| Odor evaluation at high temperature | B | A | A | A | AA | AA | AA | A | A | D | D |

### [Odor Evaluation at High Temperature]

The cosmetics were stored at 60°C for 1 week, and then 10 expert panelists performed sensory evaluation and judged in accordance with the following criteria. The obtained results are as shown in Table 1.
AA: All of the 10 panelists answered that an unpleasant odor was not felt.
A: One or more and 2 or less of the 10 panelists answered that an unpleasant odor was felt.
B: Three or more and 4 or less of the 10 panelists answered that an unpleasant odor was felt.
C: Five or more and 6 or less of the 10 panelists answered that an unpleasant odor was felt.
D: Seven or more of the 10 panelists answered that an unpleasant odor was felt.

## Claims

1. A cosmetic comprising:
(A) a Thuja orientalis seed extract;
(B) a cyclic carboxamide derivative or a salt of the cyclic carboxamide derivative the cyclic carboxamide derivative represented by Formula (1): 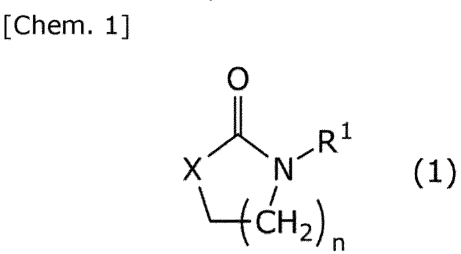 wherein
R¹ represents a hydrocarbon group having 1 to 6 carbon atoms optionally substituted with a hydroxy group or R¹ represents a hydrogen atom,
X represents -CH₂- or -N(R²)- wherein R² represents a hydrocarbon group having 1 to 6 carbon atoms optionally substituted with a hydroxy group or R² represents a hydrogen atom, and
n is an integer of 1 to 3; and
(C) water.

2. The cosmetic according to claim 1, wherein
in Formula (1) of the component (B),
R¹ represents a hydroxyalkyl group having 1 to 3 carbon atoms,
X represents -CH₂- or -NH-, and
n is 1.

3. The cosmetic according to claim 1 or 2, wherein the component (B) is 1-(2-hydroxyethyl)-2-imidazolidinone.

4. The cosmetic according to claim 1 or 2, wherein an amount of the component (B) blended is 0.01 to 8 mass% with respect to a total amount of the cosmetic.

5. The cosmetic according to claim 1 or 2, wherein the component (A) is a hydrolyzed Thuja orientalis seed extract.

6. The cosmetic according to claim 1 or 2, wherein an amount of the component (A) blended is 0.01 to 3 mass% with respect to the total amount of the cosmetic.

7. The cosmetic according to claim 1 or 2, further comprising a water-soluble thickener.
